# EUROPEAN PATENT SPECIFICATION

(11) **EP 1 993 656 B1**
(45) Date of publication and mention of the grant of the patent: **18.05.2016**
(21) Application number: 07715901.0
(22) Date of filing: 01.03.2007
(51) Int. Cl.: A61M 35/00, A45D 34/04

(54) **DEVICE FOR APPLYING A SPREADABLE COMPOSITION TO A PART OF THE HUMAN OR ANIMAL BODY**
VORRICHTUNG ZUM AUFBRINGEN EINER STREICHBAREN ZUSAMMENSETZUNG AUF EINEN TEIL EINES MENSCHLICHEN ODER TIERISCHEN KÖRPERS
DISPOSITIF POUR APPLIQUER UNE COMPOSITION POUVANT ETRE ETALEE SUR UNE PARTIE DU CORPS D'UN ETRE HUMAIN OU D'UN ANIMAL

(30) Priority: 02.03.2006 NL 2000018
(43) Date of publication of application: 26.11.2008
(73) Proprietor: YouMedical Brands B.V., 1059 AT Amsterdam (NL)
(72) Inventor: HERWEIJER, Jan, Philip, NL-1071 XK Amsterdam (NL); VAN DULLEMEN, Marlies, NL-2314 EN Leiden (NL); DOUCET-LIEM, Eleonora, NL-2312 WH Leiden (NL); WITTE, Jan, Hendricus, NL-1111 BN Aerdenhout (NL)
(74) Representative: Eveleens Maarse, Pieter
(86) International application number: PCT/NL2007/050083
(87) International publication number: WO 2007/100252

(56) References cited:
- EP-A1- 1 205 126
- WO-A-99/38561
- WO-A-99/49797
- US-A- 5 490 736

## Description

The invention relates to a device for applying a spreadable composition to a part of the human or animal body, wherein the device comprises a container containing the spreadable composition, an applicator for the spreadable composition and means for urging the spreadable composition out of the container and into contact with the applicator. Such a device is disclosed in the WO 9949797. A spreadable composition is understood to mean for instance an ointment, a gel or a cream. Such a device is known in the form of the tube and a wad of cotton, wherein the tube comprises a spreadable composition. By squeezing the tube a quantity of the spreadable composition can be placed on the wad of cotton and then be applied to a part of the body.

The application of the spreadable substance to the wad of cotton is usually accompanied by messiness; the use of this prior art device requires the skill necessary to avoid messiness.

In order to obviate the above stated problems the present invention provides positioning means connected to the container for positioning the applicator in a position in which composition urged out of the container can be brought into contact with the applicator.

As a result of these measures the process of arranging the spreadable substance on the applicator can be much better controlled, thus avoiding messiness.

The same effects are achieved by a method for applying a spreadable composition to a part of the human or animal body, comprising of carrying the spreadable composition out of a container and into contact with an applicator, and applying the spreadable composition from the applicator onto the part of a body, wherein the applicator is placed by positioning means connected to the container into a position in which composition urged from the container can be brought into contact with the applicator.

According to a preferred embodiment as further disclosed in claim 1 the container is provided with an outflow opening and the positioning means are adapted to position the applicator in a position in the vicinity of the outflow opening, in the direction of movement of the spreadable composition leaving the outflow opening.

These measures avoid the use of guide means for guiding the spreadable composition from the outflow opening to the applicator, which simplifies the structure of the device and greatly limits cleaning operations. The importance of cleaning is noted here; the applicator is usually used on and in parts of the body, wherein the danger of infection is considerable; being able to easily clean the applicator and those parts of the container which come into contact with the applicator is of the greatest importance. This measure also makes it possible for the applicator to be used more than once.

Yet another preferred embodiment provides the measure that the container is a container of the type whose internal volume can be reduced by external forces acting on the container. The process of applying the spreadable composition to the applicator can hereby be controlled better.

A simple and inexpensive preferred embodiment provides the measure that the container is formed by a tube.

In order to simplify the process of arranging the spreadable composition on the applicator for the user, which is for instance important for users with limitations, it is attractive when the container has a rigid outer wall and when the container comprises a propellant gas in addition to the spreadable composition.

A technically optimal structure is obtained when the container is provided with an internal movable wall, on one side of which the spreadable composition is present and on the other side of which the propellant gas is present. This measure makes the choice of a propellant gas easier.

Yet another preferred embodiment provides the measure that the container is provided with a valve which can be operated by the applicator positioned by the positioning means. Operation is greatly simplified hereby and the chance of messiness reduced.

Messiness when arranging the spreadable substance on the applicator is precluded when the valve can only be operated by an applicator positioned by the positioning means.

In order to prevent the spreadable composition from being absorbed into the applicator, which would be possible with the use of a applicator manufactured from a porous material such as wadding or foam, it is attractive when the applicator is provided with a core with a closed, i.e. non-porous, outer wall, this core being adapted for contact with the spreadable composition. The closed outer wall of the core will after all prevent the spreadable composition from being drawn into the applicator and no longer being applied at the desired location. The closed surface of the applicator moreover provides the option of cleaning and re-use thereof.

A specific embodiment provides the measure that the core has a first and second part of a mutually different form, wherein both parts can be placed in the positioning means and both parts are adapted to come into contact with the spreadable composition. The use of parts of mutually differing form provides the option of making a choice as to the part on which the spreadable substance is arranged. The user can let this choice depend on the nature of the location on the body where the spreadable substance must be applied, such as a body cavity. The spreadable substance is then arranged on the first part while the other part is used to grip the core.

The construction of the core is simplified when the core is rotation-symmetrical.

The options for use of the applicator increase when a sleeve extending around the core is also arranged in addition to the core. This sleeve can then be used to hold the spreadable substance fast between the core and the sleeve during transport between the container and the skin. For this purpose the sleeve is preferably formed such that it encloses a space between the sleeve and the core. This space can then be filled with the spreadable substance. The sleeve can herein be arranged around the first or the second part of the core, although the sleeve can also be arranged around both parts of the core.

These effects are likewise achieved by a method wherein the spreadable composition is carried out of the container into a space formed in the applicator.

According to a subsequent embodiment, the sleeve is provided with openings. These openings are used to apply the spreadable composition. The applicator is hereby particularly suitable for applying the spreadable composition in body cavities. This is because the spreadable composition exits not only at the tip but also on the sides, so that the composition is well distributed over the side surfaces of the body cavities.

These advantages are also achieved in a method wherein the spreadable substance is carried at least partially through the filling opening into and out of the space.

When the container is a container provided with a propellant gas, use is preferably made of a method wherein the spreadable composition flows out of the container into the space formed in the applicator through operation of a valve of the container by the applicator.

In order to facilitate filling and emptying of the space it is attractive for the sleeve to be movable in axial direction relative to the core. This is because it hereby becomes possible, in combination with an appropriate design of the core and the sleeve, to vary the volume of the space between the core and the sleeve, which can be used to urge out the spreadable substance, particularly when the spreadable substance is being applied.

The same effect is achieved by a method wherein the spreadable composition is urged from the applicator to the body by reducing the volume of the cavity formed in the applicator.

In order to be able to drive or operate the relative movement of the sleeve and the core by hand, it is attractive when the sleeve is provided with a collar extending substantially in radial direction. This collar can then be gripped with the fingers.

Urging the spreadable substance out of the space between the core and the cavity is facilitated when the sleeve is adapted to be closed internally by the core over a substantial part of its path of movement. The core then functions as piston inside the sleeve functioning as cylinder.

The structure of the applicator according to the above stated measures is simplified when the interior of the sleeve is cylindrical. The core can hereby slide reciprocally in the sleeve in the manner of a piston in a cylinder.

For some applications it can be attractive to give the sleeve a conical form on its outside. It is then possible to give the inner side of the sleeve a cylindrical form so that the above stated measures can be applied, but it is also possible to make use of a largely conical cavity, wherein the sleeve is manufactured from flexible material such that it can be deformed such that it is sealed by the cylindrical core.

Filling of the applicator is moreover facilitated when the sleeve is provided on its end remote from the core with a filling opening for filling the space between the core and the sleeve with the spreadable composition. In order to prevent a large part of the spreadable composition flowing out via this filling opening during application thereof it is attractive here when the filling opening is not too large.

It is however also possible for the core to be hollow and for the core to be provided with a filling opening for filling the space between the core and the sleeve with the spreadable composition. The hollow space of the core can herein be placed into connection with the valve of the container. It is then attractive when the valve can be operated by the core, for instance in that the core engages the valve.

The filling opening is of course adapted to fill the space between core and sleeve, but this filling opening can also be used to urge out the spreadable composition during application thereof.

In order to facilitate the dosaging of the spreadable composition a scale division is arranged on the core. This indicator can be used when filling the space. The core does after all protrude from the sleeve, so that a scale division arranged on the core is easy to read.

A method wherein the volume of the space is read during filling of the space between core and sleeve also results in these advantages.

When the sleeve and the core are adapted to displace the core relative to the sleeve when the space between the core and the sleeve is filled through the filling opening arranged in the sleeve, the core can be moved outward in the manner of a piston during filling so as to provide space for the spreadable composition.

The filling procedure of the applicator is greatly simplified when the sleeve is adapted to operate the valve of the container. In order to fill the applicator it is then only necessary to press the sleeve with the fingers until a sufficient quantity of the spreadable composition has entered the applicator.

The transfer between the sleeve and the valve is simplified when the sleeve is adapted to engage on an operating element of the valve.

In order to prevent lateral movements of the applicator it is attractive for the positioning means to be adapted to fix the sleeve by means of a snap connection.

The operation of the filling process is facilitated when the snap connection is adapted to allow a movement of the sleeve in axial direction relative to the positioning means.

It is of course possible to design both the core and the sleeve, or each individually, for once-only use. It is however attractive to design at least one of the sleeve or the core for repeated use; both parts, but in particular the core, can be readily cleaned after use. In some cases it can however be attractive to replace the sleeve after each use since this cannot be cleaned quite so well due to the presence of the openings.

When the cavity in the sleeve is filled and the applicator must be removed in its entirety, there is a danger, due to the closure between the valve and the sleeve, of an underpressure being created in the sleeve which forces the content of the cavity outward while the core moves inward. In order to prevent this problem a specific preferred embodiment proposes to arrest the core in the sleeve. Although an underpressure is still developed here, it is only developed until the filling opening has been released from the valve.

In order to perform this arrest as easily as possible, it is recommended that at least a part of the sleeve is compressible, and that the core is arrested in the sleeve when the sleeve is compressed.

This compressibility can be achieved in easy manner by providing the sleeve with notches on either side of its cavity.
An important use of the device relates to the application of the spreadable composition in a body cavity, such as the pharynx, the ears, the nostrils, the armpits or the vagina.

The most attractive use relates here to the application of the spreadable substance in the anus.

The invention also relates to a container which is adapted as a container in a device according to any of the foregoing claims, and it also relates to an applicator which is adapted as an applicator in a device according to any of the foregoing claims.

The present invention will now be elucidated with reference to the accompanying figures, in which:
Fig. 1A is a cross-sectional view of a first embodiment of the invention with an administering core in a first position;
Fig. 1B is a cross-sectional view of the embodiment shown in figure 1A with an administering core in a second position;
Fig. 2 is a cross sectional view of a second embodiment;
Fig. 3A-3F are cross-sectional views of a third embodiment in various positions;
Fig. 4 is a cross-sectional view of a variant of the third embodiment; and
Fig. 5 is a cross-sectional view of a fourth embodiment.

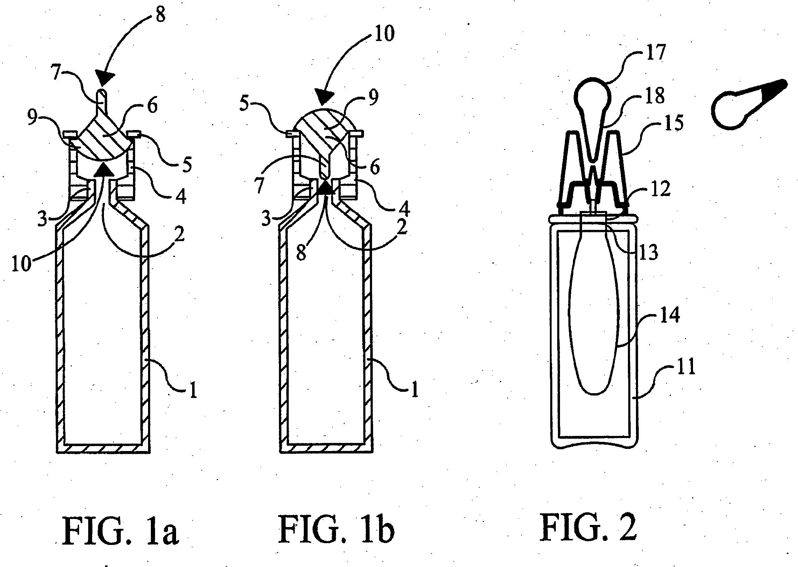

Figure 1 shows a tube 1 which serves as a container and which is filled with a spreadable substance such as a cream or an ointment. Tube 1 is provided in the usual manner with an opening 2, which is enclosed by an annular element 3 which is provided on its outside with a screw thread. A positioning element in the form of a cylinder 4 is screwed onto the screw thread. The positioning element is provided for this purpose with an opening provided with internal screw thread. On its side remote from the opening the positioning element is provided with a positioning ring 5. This positioning ring 5 serves for positioning an applicator 6.

The applicator 6 shown in figure 1 comprises a substantially cylindrical part 7 which is provided with a rounded top 8 and a substantially conical part 9, which is provided with a round base 10 and the top of which transposes into cylindrical part 7. Applicator 6 is herein adapted to apply the spreadable material in the form of an ointment or a cream to the body. The form of the applicator depends on the place on the body where the ointment or cream is applied, such as a body cavity.

In use of this embodiment the user places the applicator with the desired administering side on the positioning device, wherein the position of the applicator is determined by positioning ring 5. The user then presses tube 1, whereby a reduction in the volume of the content of the tube occurs and spreadable composition flows out of opening 2 of tube 1, and the composition enters the interior of the positioning device and comes into contact with the spherical surface 10 of applicator 6. The applicator is then removed and used to apply the ointment or cream at the desired location on or in the body.

As shown in figure 1B, it is however also possible to use the other side of applicator 6. Use can also be made here of positioning ring 5 to position applicator 6 in its other position. Cylindrical part 7 of the applicator herein comes into contact with the ointment or cream to be applied. This latter position of applicator 6 is particularly suitable for applying the ointment or cream in a body cavity such as the anus.

Use can be made as container, instead of the above elucidated tube 1, of a container with rigid walls which is filled with - in addition to the spreadable composition - a propellant gas for propelling the spreadable substance to the outside. Such a container 11 is shown in figure 2. This container 11 is provided with rigid walls 12 and the container is sealed on its top side in per se known manner by a top wall 12 in which a controllable valve 13 is placed. This embodiment is provided with an internal wall 14 of flexible material which serves as separation between the propellant gas, which is situated on the outside of this substantially bag-like wall 14, and the spreadable composition situated on the inside. It must be ensured here that the inner side of wall 14 connects to the valve so that when the valve is operated the spreadable composition flows to the outside. For positioning of the applicator use is made of an attachment 15 which functions as positioning element and which is mounted releasably on the top wall of the container. This attachment is adapted to position both the spherical part 17 and the sharp part 18 of applicator 16. The releaseability serves to enable cleaning of the attachment.

It is important here that the positioning takes place such that sufficient space remains for the cream or the ointment to be able to reach the applicator and enter into contact therewith. A certain degree of play may be necessary for this purpose. The sealed or impermeable character of the applicator is further of importance here, on the one hand to achieve that the cream or the ointment does not penetrate into the applicator, but moreover to enable proper cleaning of the applicator in the case of repeated use.

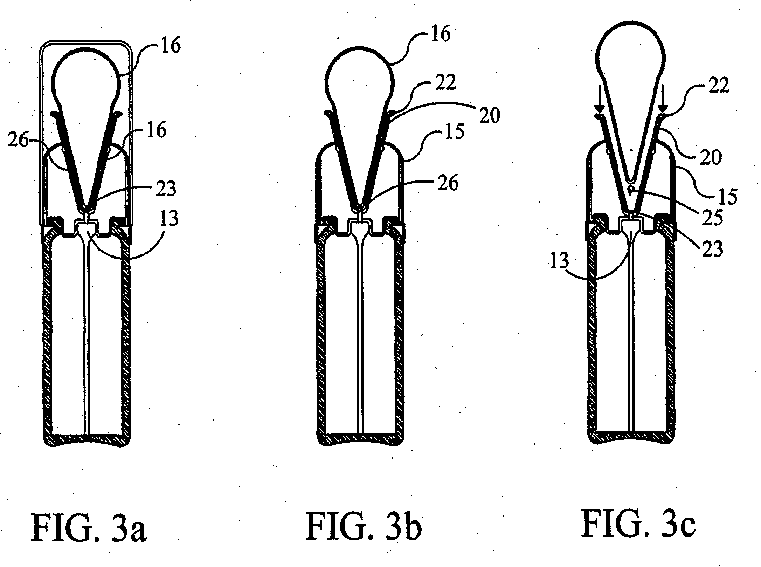

The embodiment shown in figures 3A-3F differs from the foregoing embodiment in the different structure of the applicator. The applicator is essentially formed here by a core 16 which corresponds to applicator 17 as a whole according to the foregoing embodiment. Around this core 16 is arranged a sleeve 20 which comprises a substantially conical part 21, which is provided at its wide end with a collar 22. The underside of the conical part is provided with a filling opening 23. A number of openings 24 are further placed in conical part 21 in the vicinity of this filling opening 23. The choice of material for sleeve 20 and core 16 is herein such that core 16 can slide over a limited path inside sleeve 20, wherein in the manner of a piston the core closes a cylinder over this path. A space 25 with a variable volume is thus created between the core and the sleeve. It will be apparent that such a variable volume can also be obtained in other ways, for instance by making use of cylindrical elements. Rigid materials can then suffice for the core and the sleeve.

The container according to this embodiment differs from the container elucidated with reference to figure 2 in the absence of the flexible wall inside the container. An adapter 26 is also arranged in attachment 15. This adapter 27 serves for better guiding of the applicator when it is placed on the container. On its top side the adapter 26 is mounted in attachment 15 for movement in axial direction of the otherwise cylindrical container 11. On its underside the adapter 26 is connected to valve 13, this in a manner such that valve 13 of the container is operated when adapter 26 is moved downward. Just as attachment 15, adapter 26 is also removable to enable cleaning. For this purpose the adapter is provided on its outside with a thickened portion 33 which extends all round and engages releasably under a curved part of attachment 15. A removable cap 27 is arranged for protection of the parts placed on container 11.

The operation of this device will now be described. Starting from the situation shown in figure 3A in which cap 27 is situated on container 11, cap 27 is first removed. The situation shown in figure 3B is then obtained. Applicator 16, 20 is here accessible.

By pressing on collar 22 of sleeve 20 the assembly of sleeve 20, core 16 and adapter 27 is moved downward. This situation is shown in figure 3C. Valve 13 is operated by moving adapter 26 downward, whereby valve 13 opens and spreadable composition leaves valve 13 from the container. During this process the spreadable composition fills space 25 between core 16 and sleeve 20, wherein core 16 is forced upward. When sufficient spreadable composition is present in space 25 the user stops pressing sleeve 20.

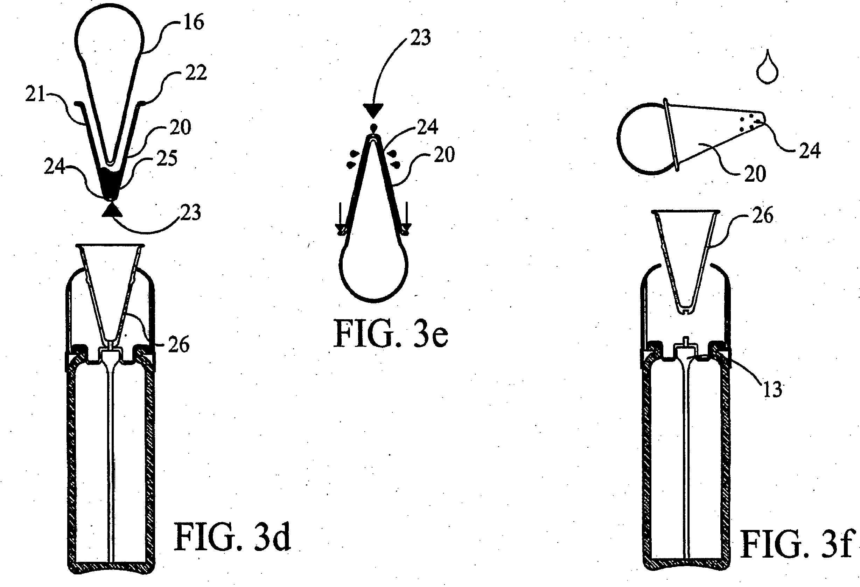

The applicator in the form of the assembly of sleeve 20 and core 16 can then be taken out, as shown in figure 3D. The removed assembly can subsequently be brought to the location on the body or into the body cavity where the spreadable composition must be applied. By moving sleeve 20 to the thickened part 17 of core 16 the space 25 between sleeve 20 and thickened part 17 is reduced, whereby the material present in this space 25 is forced outward through openings 23, 24. This is shown in figure 3E.

Applicator 16, 20 can then be cleaned, as can the adapter, for instance under the hot tap as shown in figure 3F. The components can subsequently be re-placed on container 11.

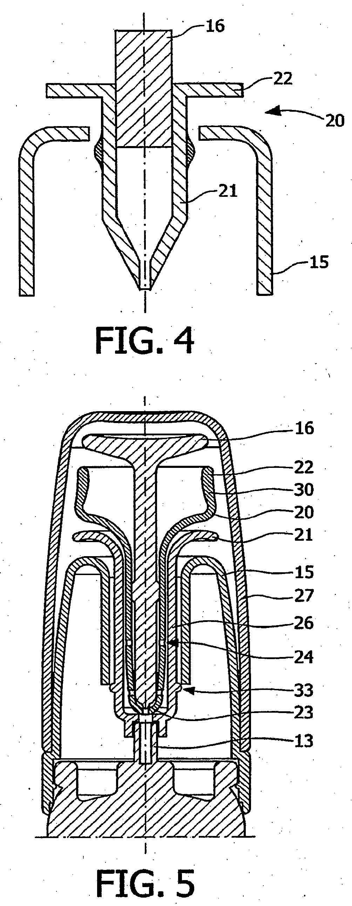

Figure 4 shows a variant of the embodiment shown in figures 3A-3F in more detail. This variant differs due to the presence of a cylindrical core 16 which is movable in the cylindrical space in sleeve 20, wherein a good seal between core 16 and sleeve 20 is ensured. This is in contrast to the embodiment shown in figures 3A-3F, wherein the seal is obtained by deforming the substantially conical sleeve 20. In the present embodiment sleeve 20 is conical only in the vicinity of filling opening 23. The core is further provided with a scale division 28. It is hereby possible to properly determine the dosage of the spreadable composition.

In the above elucidated embodiments there is always a sleeve in which a piston is displaceable for the purpose of obtaining a space with a variable volume. It is otherwise also possible to obtain a variable volume by making use of a bellows-like applicator, wherein a hollow core is used and wherein the cavity of the core connects to a bellows with a variable volume. The tip of the core can here also be used to operate the valve of the container. When the valve is operated, the bellows will be filled with spreadable composition, and when the volume of the bellows is reduced the composition will flow out of openings arranged in the core.

The fourth embodiment shown in figure 5 differs from the embodiment according to figure 3 in the cylindrical design of the components. The applicator is hereby provided with a cylindrical space, and core 16 will move inside sleeve 20 in the manner of a piston so that the volume of space 25 is reduced by moving core 16 toward sleeve 20, and the spreadable material present in the space is urged out through openings 23, 24 present in the sleeve. In this embodiment sleeve 20 further transposes directly into collar 30 so that the contamination of the space between collar 30 and core 20 present in the previous embodiment is prevented.

It is also possible to arrange in sleeve 21 two grooves, not shown in the drawing, which extend in axial direction of collar 22 to a position a certain distance from the underside of sleeve 21. As a result of the presence of the grooves the upper part of sleeve 21 can be compressed. This compressibility is used to fixedly clamp core 16 after filling of the applicator during removal from the valve. There is after all then the danger of the content of the space between sleeve 21 and core 16 being suctioned away. A reduction in volume is prevented by fixedly clamping core 16. In order to facilitate the clamping of core 16 the collar 22, which is of course also divided into two parts by the grooves, is connected to a cylindrical part 30 placed under collar 22. This cylindrical part 30 is also divided into two parts by the grooves so as to enable compressing of the two resulting pieces of part 30.

It will be apparent that the measures shown in diverse embodiments can be combined. Numerous variations of the embodiments shown here can of course be applied within the scope of the invention.

## Claims

1. Device for applying a spreadable composition to a part of the human or animal body, wherein the device comprises:
- a container (1, 11) containing the spreadable composition;
- an applicator (6, 16) for the spreadable composition; and
- means (12, 13, 14) for urging the spreadable composition out of the container (1, 11) and into contact with the applicator (6,16),
- positioning means (4, 5, 15) connected to the container (1, 11) for positioning the applicator (6, 16) in a position in which composition urged out of the container can be brought into contact with the applicator;
- wherein the container (1, 11) is provided with an outflow opening (2, 13) ; and
- wherein the positioning means (4, 5, 15) are adapted to position the applicator (6, 16) in a position in the vicinity of the outflow opening (2, 13), in the direction of movement of the spreadable composition leaving the outflow opening (2, 13),
**characterized in that** the applicator (6, 16) is provided with a core (7, 9, 17, 18) with a closed outer wall, this core (7, 9, 17, 18) being adapted for contact with the spreadable composition

2. Device as claimed in claim 1, **characterized in that** a sleeve (20) is arranged around the core (17, 18), wherein the sleeve (20) is provided with openings (24) for applying the spreadable composition through these openings (24) and the sleeve (20) is movable in axial direction relative to the core (17,18).

3. Device as claimed in claim 1 or 2, **characterized in that** the sleeve (20) is adapted to be closed internally by the core (16) over a substantial part (21) of its path of movement and that the interior of the sleeve (20) is cylindrical.

4. Device as claimed in claim 3, **characterized in that** the sleeve (20) is deformable.

5. Device as claimed in claim 2, 3 or 4, **characterized in that** the sleeve (20) is provided on its end remote from the core (16, 17, 18) with a filling opening (23) for filling the space between the core (16, 17, 18) and the sleeve (20) with the spreadable composition.

6. Device as claimed in claim 5, **characterized in that** the filling opening (23) is adapted to allow the spreadable composition to flow outside when the volume of the space between the sleeve (20) and the core (16, 17, 18) is reduced.

7. Device as claimed in claim 5 or 6 **characterized in that** the sleeve (20) and the core (16, 17, 18) are adapted to displace the core (16, 17, 18) relative to the sleeve (20) when the space between the core and the sleeve is filled through the filling opening (23) arranged in the sleeve (20).

8. Device as claimed in claim 7, **characterized in that** the sleeve (20) is adapted to operate the valve (13) of the container (11).

9. Device as claimed in any of the claims 2-8, **characterized in that** the sleeve (20) is coupled replaceably to the core (16, 17, 18) and that the core (16, 17, 18) is arrestable in the sleeve (20).

10. Device as claimed in any of the foregoing claims, **characterized in that** the applicator (6, 16) is adapted to apply the spreadable composition in a body cavity.

11. Device as claimed in any of the preceding claims, **characterized in that** the container (1) is a container of the type whose internal volume can be reduced by external forces acting on the container (1).

12. Device as claimed in any of the claims 1-10, **characterized in that** the container (11) has a rigid outer wall (15) and that the container (11) comprises a propellant gas in addition to the spreadable composition and that the container (11) is provided with an internal movable wall (14), on one side of which the spreadable composition is present and on the other side of which the propellant gas is present.

13. Device as claimed in claim 12, **characterized in that** the container (1, 11) is provided with a valve (13) which can be operated by the applicator (16) positioned by the positioning means (15) .

14. Device as claimed in claim 13, **characterized in that** the valve (13) can only be operated by an applicator (16) positioned by the positioning means (15).

## Patentansprüche

1. Vorrichtung zum Aufbringen einer streichbaren Zusammensetzung auf einen Teil eines menschlichen oder tierischen Körpers, wobei die Vorrichtung umfasst:
einen Behälter (1, 11), der die streichbare Zusammensetzung enthält;
einen Applikator (6, 16) für die streichbare Zusammensetzung; und
Mittel (12, 13, 14) zum Drängen der streichbaren Zusammensetzung aus dem Behälter (1, 11) und in Kontakt mit dem Applikator (6,16),
Positionierungsmittel (4, 5, 15), die mit dem Behälter (1, 11) zum Positionieren des Applikators (6, 16) in einer Position verbunden sind, in der die Zusammensetzung, die aus dem Behälter gedrängt wird, in Kontakt mit dem Applikator gebracht werden kann;
wobei der Behälter (1, 11) mit einer Ausflussöffnung (2, 13) bereitgestellt ist; und
wobei die Positionierungsmittel (4, 5, 15) zum Positionieren des Applikator (6, 16) in einer Position in der Nähe der Ausflussöffnung (2, 13) in Bewegungsrichtung der streichbaren Zusammensetzung, welche die Ausflussöffnung (2, 13) verlässt, ausgelegt sind,
**dadurch gekennzeichnet, dass** der Applikator (6, 16) mit einem Kern (7, 9, 17, 18) mit einer geschlossenen Außenwand bereitgestellt ist, wobei dieser Kern (7, 9, 17, 18) zum Kontaktieren der streichbaren Zusammensetzung ausgelegt ist.

2. Vorrichtung nach Anspruch 1, **dadurch gekennzeichnet, dass** eine Hülse (20) um den Kern (17, 18) herum angeordnet ist, wobei die Hülse (20) mit Öffnungen (24) zum Aufbringen der streichbaren Zusammensetzung durch diese Öffnungen (24) ausgelegt ist und die Hülse (20) in axialer Richtung in Bezug auf den Kern (17, 18) bewegt werden kann.

3. Vorrichtung nach Anspruch 1 oder 2, **dadurch gekennzeichnet, dass** die Hülse (20) zum internen Verschließen durch den Kern (16) über einen wesentlichen Teil (21) ihrer Bewegungsbahn ausgelegt ist, und dadurch, dass der Innenraum der Hülse (20) zylindrisch ist.

4. Vorrichtung nach Anspruch 3, **dadurch gekennzeichnet, dass** die Hülse (20) verformbar ist.

5. Vorrichtung nach Anspruch 2, 3 oder 4, **dadurch gekennzeichnet, dass** die Hülse (20) an ihrem vom Kern (16, 17, 18) entfernten Ende mit einer Einfüllöffnung (23) zum Füllen des Raums zwischen dem Kern (16, 17, 18) und der Hülse (20) mit der streichbaren Zusammensetzung bereitgestellt ist.

6. Vorrichtung nach Anspruch 5, **dadurch gekennzeichnet, dass** die Einfüllöffnung (23) ausgelegt ist, damit die streichbare Zusammensetzung außerhalb fließen kann, wenn das Volumen des Raums zwischen der Hülse (20) und dem Kern (16, 17, 18) reduziert wird.

7. Vorrichtung nach Anspruch 5 oder 6, **dadurch gekennzeichnet, dass** die Hülse (20) und der Kern (16, 17, 18) zum Verschieben des Kerns (16, 17, 18) in Bezug auf die Hülse (20) ausgelegt sind, wenn der Raum zwischen Kern und Hülse durch die Einfüllöffnung (23), die an der Hülse (20) angeordnet ist, gefüllt wird.

8. Vorrichtung nach Anspruch 7, **dadurch gekennzeichnet, dass** die Hülse (20) zum Betreiben des Ventils (13) des Behälters (11) ausgelegt ist.

9. Vorrichtung nach einem der Ansprüche 2 bis 8, **dadurch gekennzeichnet, dass** die Hülse (20) austauschbar mit dem Kern (16, 17, 18) gekoppelt ist und dass der Kern (16, 17, 18) in der Hülse (20) einrasten kann.

10. Vorrichtung nach einem der vorhergehenden Ansprüche, **dadurch gekennzeichnet, dass** der Applikator (6, 16) zum Aufbringen der streichbaren Zusammensetzung in eine Körperhöhle ausgelegt ist.

11. Vorrichtung nach einem der vorhergehenden Ansprüche, **dadurch gekennzeichnet, dass** der Behälter (1) ein Behälter des Typs ist, dessen Innenvolumen durch äußere Kräfte, die auf den Behälter (1) einwirken, reduziert werden kann.

12. Vorrichtung nach einem der Ansprüche 1 bis 10, **dadurch gekennzeichnet, dass** der Behälter (11) eine starre Außenwand (15) aufweist und dass der Behälter (11) ein Treibgas zusätzlich zu der streichbaren Zusammensetzung umfasst und dass der Behälter (11) mit einer internen beweglichen Wand (14) bereitgestellt ist, auf deren einer Seite die streichbare Zusammensetzung vorhanden ist und auf deren anderer Seite das Treibgas vorhanden ist.

13. Vorrichtung nach Anspruch 12, **dadurch gekennzeichnet, dass** der Behälter (1, 11) mit einem Ventil (13) bereitgestellt ist, das durch den Applikator (16) betrieben werden kann, der von dem Positionierungsmittel (15) positioniert wird.

14. Vorrichtung nach Anspruch 13, **dadurch gekennzeichnet, dass** das Ventil (13) nur durch einen Applikator (16) betrieben werden kann, der von dem Positionierungsmittel (15) positioniert wird.

## Revendications

1. Dispositif pour appliquer une composition pouvant être étalée sur une partie du corps d'un être humain ou d'un animal, dans lequel le dispositif comprend:
- un récipient (1, 11) contenant la composition à étaler;
- un applicateur (6, 16) pour la composition à étaler; et
- des moyens (12, 13, 14) pour pousser la composition à étaler hors du récipient (1, 11) et en contact avec l'applicateur (6, 16),
- des moyens de positionnement (4, 5, 15) connectés au récipient (1, 11) pour positionner l'applicateur (6, 16) dans une position dans laquelle la composition poussée hors du récipient peut être amenée en contact avec l'applicateur;
- dans lequel le récipient (1, 11) comporte une ouverture d'écoulement de sortie (2, 13); et
- dans lequel les moyens de positionnement (4, 5, 15) sont aptes à positionner l'applicateur (6, 16) dans une position dans le voisinage de l'ouverture d'écoulement de sortie (2, 13), dans la direction de déplacement de la composition à étaler qui sort par l'ouverture d'écoulement de sortie (2, 13),
**caractérisé en ce que** l'applicateur (6, 16) est pourvu d'un noyau (7, 9, 17, 18) présentant une paroi extérieure fermée, ledit noyau (7, 9, 17, 18) étant apte à entrer en contact avec la composition à étaler.

2. Dispositif selon la revendication 1, **caractérisé en ce qu'**un manchon (20) est agencé autour du noyau (17, 18), dans lequel le manchon (20) comporte des ouvertures (24) pour appliquer la composition à étaler à travers ces ouvertures (24), et le manchon (20) est mobile dans la direction axiale par rapport au noyau (17, 18).

3. Dispositif selon la revendication 1 ou 2, **caractérisé en ce que** le manchon (20) est apte à être fermé intérieurement par le noyau (16) sur une partie substantielle (21) de son chemin de déplacement, et **en ce que** l'intérieur du manchon (20) est cylindrique.

4. Dispositif selon la revendication 3, **caractérisé en ce que** le manchon (20) est déformable.

5. Dispositif selon la revendication 2, 3 ou 4, **caractérisé en ce que** le manchon (20) est pourvu sur son extrémité distante du noyau (16, 17, 18) d'une ouverture de remplissage (23) pour remplir l'espace entre le noyau (16, 17, 18) et le manchon (20) avec la composition à étaler.

6. Dispositif selon la revendication 5, **caractérisé en ce que** l'ouverture de remplissage (23) est apte à permettre à la composition à étaler de s'écouler à l'extérieur lorsque le volume de l'espace entre le manchon (20) et le noyau (16, 17, 18) est réduit.

7. Dispositif selon la revendication 5 ou 6, **caractérisé en ce que** le manchon (20) et le noyau (16, 17, 18) sont aptes à déplacer le noyau (16, 17, 18) par rapport au manchon (20) lorsque l'espace entre le noyau et le manchon est rempli à travers l'ouverture de remplissage (23) qui est prévue dans le manchon (20).

8. Dispositif selon la revendication 7, **caractérisé en ce que** le manchon (20) est apte à actionner la valve (13) du récipient (11).

9. Dispositif selon l'une quelconque des revendications 2 à 8, **caractérisé en ce que** le manchon (20) est couplé de façon remplaçable au noyau (16, 17, 18), et **en ce que** le noyau (16, 17, 18) peut être arrêté dans le manchon (20).

10. Dispositif selon l'une quelconque des revendications précédentes, **caractérisé en ce que** l'applicateur (6, 16) est apte à appliquer la composition à étaler dans une cavité corporelle.

11. Dispositif selon l'une quelconque des revendications précédentes, **caractérisé en ce que** le récipient (1) est un récipient du type dont le volume interne peut être réduit par des forces externes qui agissent sur le récipient (1).

12. Dispositif selon l'une quelconque des revendications 1 à 10, **caractérisé en ce que** le récipient (11) présente une paroi extérieure rigide (15), et **en ce que** le récipient (11) contient un gaz propulseur en plus de la composition à étaler, et **en ce que** le récipient (11) est pourvu d'une paroi mobile interne (14), sur un premier côté de laquelle la composition à étaler est présente, et sur l'autre côté de laquelle le gaz propulseur est présent.

13. Dispositif selon la revendication 12, **caractérisé en ce que** le récipient (1, 11) est muni d'une valve (13) qui peut être actionnée par l'applicateur (16) positionné par les moyens de positionnement (15).

14. Dispositif selon la revendication 13, **caractérisé en ce que** la valve (13) peut uniquement être actionnée par un applicateur (16) qui est positionné par les moyens de positionnement (15).
